# EUROPEAN PATENT APPLICATION

(11) **EP 4 046 665 A1**
(43) Date of publication of application: **24.08.2022**
(21) Application number: 22168086.1
(22) Date of filing: 08.09.2020
(51) Int. Cl.: A61L 27/36, A61L 27/46, A61L 27/50, A61L 27/54, A61L 27/56

(54) **BONE GRAFT COMPOSITION AND PREPARATION METHOD THEREFOR**

(30) Priority: 10.04.2020 KR 20200044124; 21.05.2020 KR 20200060836; 05.06.2020 KR 20200068042
(62) Divisional of application: 20195009.4
(71) Applicant: MedPark Co., Ltd., Busan 46504 (KR)
(72) Inventor: PARK, Jung Bok, 46509 Busan (KR)
(74) Representative: BCKIP

(57) **Abstract**

The present disclosure relates to a bone graft composition, and more particularly, to a bone graft composition including hydroxypropyl methylcellulose and a preparation method therefor. Moreover, the present disclosure relates to a bone graft composition that has an optimal composition ratio at which the dissolution rate of hydroxypropyl methylcellulose is excellent. In addition, the present disclosure relates to a bone graft composition containing hydroxypropyl methylcellulose in an amount that provides shape retainability, and a preparation method therefor. More specifically, the present disclosure relates to a bone graft composition containing hydroxypropyl methylcellulose in an amount that provides an optimum osmotic pressure and shape retainability, and a preparation method therefor.

## Description

### CROSS-REFERENCE TO RELATED APPLICATION(S)

This application claims priority to Korean Patent Application No. 10-2020-0044124, filed on April 10, 2020, Korean Patent Application No. 10-2020-0060836, filed on May 21, 2020, and Korean Patent Application No. 10-2020-0068042, filed on June 5, 2020, the disclosure of which is incorporated herein by reference in its entirety.

### BACKGROUND OF THE DISCLOSURE

### Technical Field

The present disclosure relates to a bone graft composition having an excellent dissolution rate of hydroxypropyl methylcellulose and excellent shape retainability, and a preparation method therefor.

### Description of the Related Art

Various materials and various methods may be used for reconstruction of defective bone. For example, bone graft materials such as bone powders, bone chips, and bone blocks may be used, or methods such as autografting, allografting, and xenografting may be used for reconstruction of defective bone.

Bone graft materials that are used for reconstruction of defective bone may be used in orthopedic surgery, neurosurgery, plastic surgery, otolaryngology, Oral and Maxillofacial Surgery, Department of Veterinary Medicine(veterinary clinic), dermatology and dentistry. For example, these materials may be used for bone defects during disc surgery to induce bone regeneration, or may also be used for implant surgery and reconstruction of oral and maxillofacial bone defects.

Meanwhile, Korean Patent No. 10-0401941 discloses technology related to a bone graft material and a preparation method therefor. When a reticular bone is used which composed of bioceramic powder and has a three-dimensionally communicating pore structure as disclosed therein is used, there may be limitations in the effect of bone graft in terms of biocompatibility, mechanical properties, toxicity, and the like.

### SUMMARY

An object of the present disclosure is to provide a bone graft composition, which includes hydroxypropyl methylcellulose having a dissolution rate suitable for bone formation and has excellent shape retainability, and a preparation method therefor.

Another object of the present disclosure is to provide a bone graft composition containing hydroxypropyl methylcellulose suitable for bone formation, specifically a bone graft composition and a bone graft solution, which form an optimum osmotic pressure with another solution.

One embodiment of the present disclosure provides a bone graft composition containing a bone graft material and hydroxypropyl methylcellulose, wherein the hydroxypropyl methylcellulose reaches a dissolution rate of 50% or more within 48 hours.

One embodiment of the present disclosure provides a bone graft composition wherein the bone graft composition contains the hydroxypropyl methylcellulose in an amount of 0.3 to 3 parts by weight based on 1 part by weight of the bone graft material.

One embodiment of the present disclosure provides a bone graft composition wherein the bone graft material is a natural bone graft material including a porous structure.

One embodiment of the present disclosure provides a method for preparing a bone graft composition, the method including steps of: (1) preparing a bone morphogenetic protein solution by mixing a solvent and a bone morphogenetic protein; (2) adsorbing the bone morphogenetic protein onto graft material powder by mixing the bone morphogenetic protein and the graft material powder; (3) mixing and stirring the graft material powder having the bone morphogenetic protein adsorbed thereon and hydroxypropyl methylcellulose powder to obtain a mixture, and forming a gel from the mixture such that the dissolution rate of the hydroxypropyl methylcellulose powder reaches 50% or more within 48 hours; and (4) forming a structure containing a plurality of pores by freeze-drying the gel under vacuum.

One embodiment of the present disclosure provides a method for preparing a bone graft composition, wherein the bone morphogenetic protein is at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto.

One embodiment of the present disclosure provides a method for preparing a bone graft composition, wherein the concentration of the bone morphogenetic protein in the bone morphogenetic protein solution may be 0.05 to 0.15 mg/ml.

One embodiment of the present disclosure provides a method for preparing a bone graft composition, wherein the pH of the bone morphogenetic protein solution is adjusted to 4.6 to 5 using phosphate buffer saline.

One embodiment of the present disclosure provides a method for preparing a bone graft composition, wherein the volume ratio between the graft material powder having the bone morphogenetic protein adsorbed thereon and the hydroxypropyl methylcellulose powder in step (3) is 1:0.2 to 1:0.8.

One embodiment of the present disclosure provides a method for preparing a bone graft composition, wherein the method furthers include a step of sterilizing the bone graft composition by ethylene oxide gas or gamma-ray irradiation.

One embodiment of the present disclosure provides a method for preparing a bone graft composition, wherein the concentration of the ethylene oxide gas is 450 to 1,200 mg/l, or the dose of the gamma-ray irradiation is 10 to 25 kGy.

One embodiment of the present disclosure provides a bone graft composition having shape retainability of 50 or more, wherein the shape retainability is defined as a value obtained by dividing a maximum breaking force (Nmax) by a short-axis change rate, in which the maximum breaking force is a force at which a change in the shape of a sphere-shaped material occurs, and the short-axis change rate is the ratio of the reduction in short-axis length of the sphere-shaped material, measured after the change in the shape occurred, to the diameter of the sphere-shaped material.

The bone graft composition contains 1 part by weight of a bone graft material mixed with 0.3 to 3 parts by weight of hydroxypropyl methylcellulose.

One embodiment of the present disclosure provides a bone graft composition having excellent shape retainability, wherein the porous bone graft material is a natural bone graft composition.

One embodiment of the present disclosure provides a method for preparing a bone graft composition having excellent shape retainability, the method including steps of:
(1) preparing a bone morphogenetic protein solution by mixing a solvent and a bone morphogenetic protein; (2) adsorbing the bone morphogenetic protein onto graft material powder by mixing the bone morphogenetic protein and the graft material powder; (3) mixing and stirring the graft material powder having the bone morphogenetic protein adsorbed thereon and hydroxypropyl methylcellulose powder to form a gel that imparts ; and (4) forming a structure containing a plurality of pores by freeze-drying the gel under vacuum.

One embodiment of the present disclosure provides a method for preparing a bone graft composition having excellent shape retainability, wherein the bone morphogenetic protein is at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto.

One embodiment of the present disclosure provides a method for preparing a bone graft composition having excellent shape retainability, wherein the concentration of the bone morphogenetic protein in the bone morphogenetic protein solution is 0.05 to 0.15 mg/ml.

One embodiment of the present disclosure provides a method for preparing a bone graft composition having excellent shape retainability, wherein the pH of the bone morphogenetic protein solution is adjusted to 4.6 to 5 using phosphate buffer saline.

One embodiment of the present disclosure provides a method for preparing a bone graft composition having excellent shape retainability, wherein the volume ratio between the graft material powder having the bone morphogenetic protein adsorbed thereon and the hydroxypropyl methylcellulose powder in step (3) is 1:0.2 to 1:0.6.

One embodiment of the present disclosure provides a method for preparing a bone graft composition having excellent shape retainability, wherein the method further includes a step of sterilizing the bone graft composition by ethylene oxide gas or gamma-ray irradiation.

One embodiment of the present disclosure provides a method for preparing a bone graft composition having excellent shape retainability, wherein the concentration of the ethylene oxide gas is 450 to 1,200 mg/l, or the dose of the gamma-ray irradiation is 10 to 25 kGy.

One embodiment of the present disclosure provides a bone graft composition with which saline reaches an osmotic pressure of 104 to 112% within 12 to 48 hours after addition to a bone graft solution, when the osmotic pressure of the saline is set to 100% as a reference value.

One embodiment of the present disclosure provides a bone graft composition wherein the bone graft solution is a mixture of 1 part by weight of a bone graft material containing hydroxypropyl methylcellulose and 0.5 to 2 parts by weight of a solvent, and the bone graft material containing hydroxypropyl methylcellulose is formed by mixing 1 part by weight of a bone graft material with 0.3 to 3 parts by weight of hydroxypropyl methylcellulose.

One embodiment of the present disclosure provides a bone graft composition wherein the bone graft material is a natural bone graft material.

One embodiment of the present disclosure provides a bone graft composition wherein the solvent is water.

One embodiment of the present disclosure provides a method for preparing a bone graft composition, the method including steps of: (1) preparing a bone morphogenetic protein solution by mixing a solvent and a bone morphogenetic protein; (2) adsorbing the bone morphogenetic protein onto graft material powder by mixing the bone morphogenetic protein and the graft material powder; (3) mixing and stirring the graft material powder having the bone morphogenetic protein adsorbed thereon and hydroxypropyl methylcellulose powder to form a gel having osmotic properties; and (4) forming a structure containing a plurality of pores by freeze-drying the gel under vacuum.

One embodiment of the present disclosure provides a method for preparing a bone graft composition, wherein the bone morphogenetic protein is at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto.

One embodiment of the present disclosure provides a method for preparing a bone graft composition, wherein the concentration of the bone morphogenetic protein in the bone morphogenetic protein solution is 0.05 to 0.15 mg/ml.

One embodiment of the present disclosure provides a method for preparing a bone graft composition, wherein the pH of the bone morphogenetic protein solution is adjusted to 4.6 to 5 using phosphate buffer saline.

One embodiment of the present disclosure provides a method for preparing a bone graft composition, wherein the volume ratio between the graft material powder having the bone morphogenetic protein adsorbed thereon and the hydroxypropyl methylcellulose powder in step (3) is 1:0.2 to 1:0.6.

One embodiment of the present disclosure provides a method for preparing a bone graft composition, wherein the method further includes a step of sterilizing the bone graft composition by ethylene oxide gas or gamma-ray irradiation.

One embodiment of the present disclosure provides a method for preparing a bone graft composition, wherein the concentration of the ethylene oxide gas is 450 to 1,200 mg/l, or the dose of the gamma-ray irradiation is 10 to 25 kGy.

One embodiment of the present disclosure provides a bone graft composition prepared by the above-described method for preparing a bone graft composition.

### BRIEF DESCRIPTION OF THE DRAWINGS

FIG. 1 is a flow diagram schematically showing a method for preparing a bone graft composition according to one embodiment of the present disclosure.
FIG. 2 shows the time-dependent residual rate of hydroxypropyl methylcellulose as a function of the content (parts by weight) thereof, calculated based on the result data (Table 1) obtained in Experimental Example 1 of the present disclosure.
FIG. 3 shows volume reduction rates depending on the content (parts by weight) of hydroxypropyl methylcellulose, calculated based on the result data (Table 2) obtained in Experimental Example 2 of the present disclosure.
FIG. 4 shows "maximum breaking force (N) of bone graft composition sphere/short-axis change rate of bone graft composition sphere" as a function of the content (parts by weight) of hydroxypropyl methylcellulose, and demonstrates the content (parts by weight) of hydroxypropyl methylcellulose for a bone graft composition having excellent shape retainability.
FIG. 5 shows result data for an Experimental Example of the present disclosure, obtained by mixing saline with bone graft solutions formed using varying amounts (parts by weight) of a solvent, and expressing the time-dependent change in osmotic pressure of the mixed saline compared to that of pure saline as osmotic ratio.

### DESCRIPTION OF SPECIFIC EMBODIMENTS

Embodiments of the present disclosure relate to a bone graft composition which may have excellent effects in terms of activation of bone formation, biocompatibility, and ease of use by containing a porous bone graft material and hydroxypropyl methylcellulose.

However, description of a portion of a particular embodiment, which overlaps with that of other embodiments, will be omitted for a clearer and more concise explanation. Even though description of that portion is omitted, the portion is not excluded from the present disclosure and the scope of rights thereof should be admitted in the same manner as that of other embodiments.

In the following description, the detailed description of publicly-known technology related to the present disclosure will be omitted when it may unnecessarily obscure the subject matter of the present disclosure. In addition, the terms used in the following description are terms defined in consideration of their functions in the present disclosure and may be changed according to the intention of a user or an operator, or according to practice. Therefore, the definitions of these terms should be determined based on the contents throughout the specification.

The technical spirit of the present disclosure is determined by the claims, and the following embodiments are merely means for efficiently explaining the technical spirit of the present disclosure to those skilled in the art to which the present disclosure pertains.

In the present disclosure, when the repeating unit, compound or resin represented by a formula includes isomers thereof, the corresponding formula representing the repeating unit, compound or resin means a representative formula that also represents the isomers.

Hereinafter, specific embodiments of the present disclosure will be described. However, these embodiments are only examples, and the present disclosure is not limited thereto.

A bone graft composition of the present disclosure includes a porous bone graft material and hydroxypropyl methylcellulose. The bone graft composition may be implanted into a bone defect, and may be used to restore the bone defect by filling the bone defect.

Hereinafter, 'implant' includes being applied into a bone defect in the state of not having rigidity or in the state of having rigidity. Applying into a bone defect in the state of having rigidity may being implanted into a bone defect after being formed the shape corresponding to the shape of the bone defect in the state of having rigidity by a shape forming device, for example 3 dimensional printer.

The bone graft material may be natural bone, for example, autogenous bone, allogeneic bone, or xenogenic bone. When the natural bone is used, it may exhibit an excellent bone formation effect, because it has excellent biocompatibility and also has good wettability and hygroscopicity due to a large number of pores contained therein. In addition, the natural bone may also be used for reconstruction of defective bone in orthopedic surgery, neurosurgery, plastic surgery, otolaryngology, Oral and Maxillofacial Surgery, Department of Veterinary Medicine(veterinary clinic), dermatology and dentistry.

In addition, the bone graft material may also be used for reconstruction of defective bone in human or animals. Hereinafter, it mainly described the usage to the dentistry, however, the usage does not limited thereto.

As the bone graft composition includes hydroxypropyl methylcellulose, the bone graft composition may have adhesion to a bone defect. At the same time, shape retainability may be imparted to the bone graft composition by the HPMC. When the bone graft composition has excellent shape retainability, even if the bone graft composition is applied to the maxilla, it may be applied to fit the bone defect without flowing down, and thus even if there is an impact due to mastication motion or the like, the bone graft composition may be prevented from being detached from the bone defect, and at the same time, the medical procedure can be more easily performed.

In order to optimize the solubility of hydroxypropyl methylcellulose, the bone graft composition according to one embodiment of the present disclosure may contain hydroxypropyl methylcellulose in an amount of 0.1 to 6 parts by weight, more preferably 0.3 to 3 parts by weight, based on 1 part by weight of the porous bone graft material. In this case, the shape retentability of the composition is further enhanced.

If the content of the hydroxypropyl methylcellulose is less than 0.3 parts by weight based on 1 part by weight of the porous bone graft material, the hydroxypropyl methylcellulose will dissolve quickly within a short time because the content thereof is low, but a problem may arise in that the hydroxypropyl methylcellulose easily dissolves out because the amount of hydroxypropyl methylcellulose combined with the bone graft material is excessively small, so that the porous bone graft material may not function as a bone graft material. On the other hand, if the content of the hydroxypropyl methylcellulose is more than 3 parts by weight based on 1 part by weight of the porous bone graft material, curing of the hydroxypropyl methylcellulose may occur due to the high content of the hydroxypropyl methylcellulose, and thus the dissolution thereof may be very slow, which may not be suitable for a bone formation rate. In addition, in some cases, if the content of the hydroxypropyl methylcellulose is more than 3 parts by weight based on 1 part by weight of the porous bone graft material, a shape in which the hydroxypropyl methylcellulose surrounds the bone graft material can be formed due to an increased volume of the hydroxypropyl methylcellulose, and hence in the humid environment in the oral cavity, the hydroxypropyl methylcellulose can absorb moisture before dissolution thereof occurs, and thus the volume of the bone graft material can increase over time. Accordingly, in order to optimize the solubility of the hydroxypropyl methylcellulose, the content of the hydroxypropyl methylcellulose may be 0.1 to 6 parts by weight, more preferably 0.3 to 3 parts by weight, based on 1 part by weight of the porous bone graft material.

As a solvent for dissolution of the hydroxypropyl methylcellulose, water, for example, may be used. The rate of dissolution formed depending on the dissolution time by hydrating the bone graft composition is expressed as %. For hydration, water is used in an amount of 1 to 1.5 parts by weight, more preferably 1.2 to 1.5 parts by weight, based on the weight of the bone graft composition that is hydrated. This amount of water is an example of the optimal amount for hydration of the bone graft composition.

A bone graft composition kit according to another embodiment of the present disclosure includes the above-described bone graft composition and a syringe containing the composition. By providing the syringe containing the bone graft composition, it is possible to ensure ease of use and significantly reduce the possibility of contamination that may occur during use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-described embodiments.

A method for preparing a bone graft composition according to still another embodiment of the present disclosure includes steps of: preparing a bone morphogenetic protein solution by adding a bone morphogenetic protein to a solvent or adding the bone morphogenetic protein to the solvent to dissolve the bone morphogenetic protein in the solvent;
mixing and stirring graft material powder having the bone morphogenetic protein adsorbed thereon and hydroxypropyl methylcellulose powder, and forming a viscous gel such that the dissolution rate of the hydroxypropyl methylcellulose powder reaches 50% or more within 48 hours; and
forming a sponge-like structure containing a plurality of pores by freeze-drying the mixture of the graft material powder and the hydroxypropyl methylcellulose powder, obtained by the mixing and stirring process, at low temperature under vacuum. The bone graft composition prepared through these steps may have excellent effects in terms of activation of bone formation, biocompatibility, and ease of use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-described embodiments.

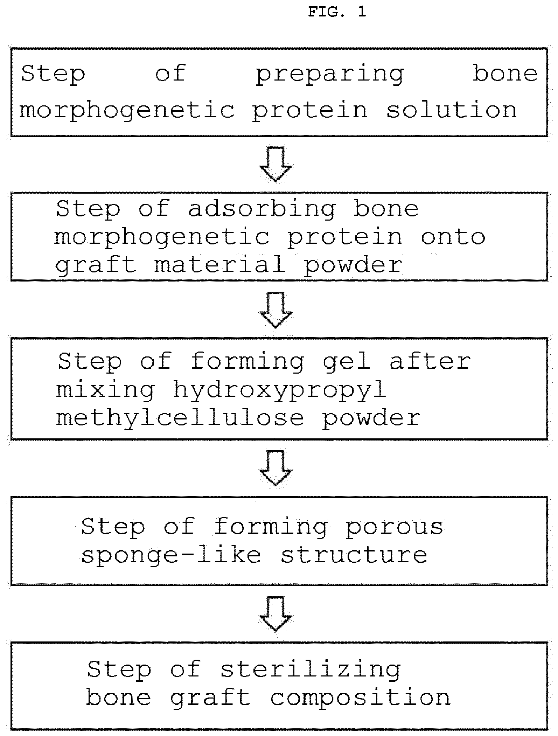

FIG. 1 is a flow diagram schematically showing a method for preparing a bone graft composition according to one embodiment of the present disclosure.

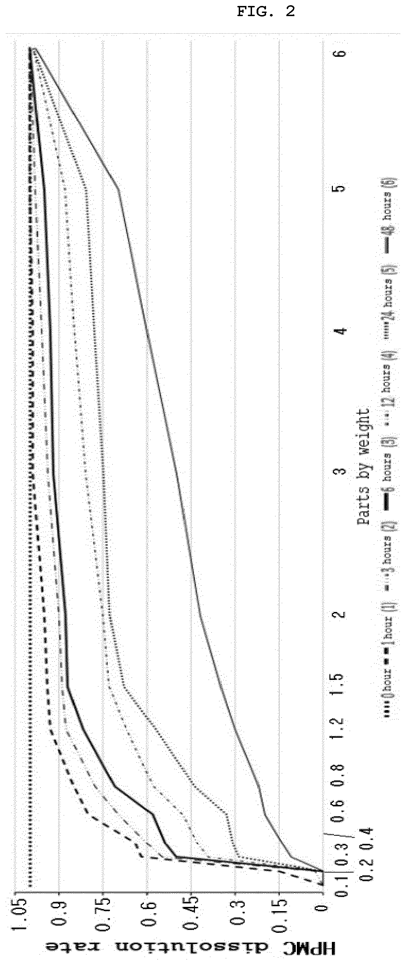

FIG. 2 shows the time-dependent residual rate of hydroxypropyl methylcellulose as a function of the content (parts by weight) thereof, calculated based on the result data (Table 1) obtained in Experimental Example 1 of the present disclosure.

First, a bone morphogenetic protein solution is prepared by dissolving a bone morphogenetic protein in a solvent. The bone morphogenetic protein solution may be prepared by adding the bone morphogenetic protein to the solvent, or adding the bone morphogenetic protein to the solvent and dissolving the bone morphogenetic protein in the solvent.

The bone morphogenetic protein may be at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto. Preferably, the bone morphogenetic protein may be rhBMP-2 in terms of the bone formation effect of the present disclosure.

According to one embodiment of the present disclosure, the concentration of the bone morphogenetic protein in the bone morphogenetic protein solution may be 0.05 to 0.15 mg/ml, preferably 0.08 to 0.12 mg/ml. When the concentration of the bone morphogenetic protein is within the above-described range, bone formation by the bone morphogenetic protein may be activated. If the concentration of the bone morphogenetic protein is less than 0.05 mg/ml, the ability of the bone morphogenetic protein to form new bone may be reduced, and if the concentration of the bone morphogenetic protein is more than 0.15 mg/ml, it may cause adverse effects.

In addition, according to one embodiment of the present disclosure, the pH of the bone morphogenetic protein solution may be, for example, 4.6 to 5. When the pH is within the above-described range, bone formation by the bone morphogenetic protein may be activated. If the pH of the bone morphogenetic protein solution is less than 4.6, the ability to form new bone may be reduced, and if the pH of the bone morphogenetic protein solution is more than 5, the ability to form new bone may be reduced. For example, the pH may be adjusted using phosphate buffer saline. When the pH is adjusted using phosphate buffer saline, the effect of forming new bone may be obtained.

Thereafter, the bone morphogenetic protein is adsorbed onto graft material powder by soaking the graft material powder with the bone morphogenetic protein solution. The previously prepared graft material powder may be soaked with the bone morphogenetic protein solution by flushing the graft material powder with the bone morphogenetic protein solution or dropping the graft material powder into the bone morphogenetic protein solution, whereby the bone morphogenetic protein may be adsorbed onto the graft material powder.

The graft material powder may be autogenous bone, allogeneic bone, or xenogenic bone. For example, the graft material powder may be prepared by placing it in a snap tube.

The average particle diameter (D50) of the graft material powder may be 200 to 5,000 µm, preferably 250 to 1,000 µm. If the average particle diameter of the powder is less than 200 µm, the graft material may be absorbed rapidly, and thus osteoconduction required for bone formation may be insufficient, and if the average particle diameter of the powder is more than 5,000 µm, precise processing for application to a patient may be difficult.

According to one embodiment of the present disclosure, the step of adsorbing the bone morphogenetic protein onto the graft material powder may include a step of adsorbing the bone morphogenetic protein using a refrigerated centrifuge.

In some cases, the bone morphogenetic protein may also be suspended in the solution. However, when the bone morphogenetic protein is adsorbed while it is rotated at high speed using a centrifuge, the bone morphogenetic protein can be prevented from being suspended in the solution, and thus the bone morphogenetic protein may be easily adsorbed onto the surface or into the pores of the graft material powder. Only when the bone morphogenetic protein is adsorbed while it is rotated at high speed, it can be prevented from being suspended again after detachment from the graft material powder. If the bone morphogenetic protein is rotated at low speed, it can be suspended, and hence cannot be easily adsorbed. Under high-speed rotation, the bone morphogenetic protein can be adsorbed quickly onto the surface or into the pores of the graft material powder.

According to one embodiment of the present disclosure, the rotational speed of the refrigerated centrifuge may be 4,000 rpm or more. When the bone morphogenetic protein is adsorbed using the centrifuge, the higher the rotational speed, the better the adsorption. For example, the rotational speed of the centrifuge may be 4,000 rpm or more, and when this rotational speed range is satisfied, the bone morphogenetic protein can be prevented from being suspended in the solution.

According to one embodiment of the present disclosure, the step of adsorbing the bone morphogenetic protein using the refrigerated centrifuge may be performed at a cold temperature of 5°C or below. As the step of adsorbing the bone morphogenetic protein using the refrigerated centrifuge is performed at a cold temperature of 5°C or below, it is possible to maximize the effect of adsorbing the bone morphogenetic protein onto the surface or into the pores of the graft material powder through rotation while preventing the denaturation of the bone morphogenetic protein that is weak to heat, by preventing the temperature of the solution from being increased due to rotation. The cold temperature may be a temperature at which the solution does not freeze. For example, the cold temperature may be 5°C or below, preferably 0.5 to 1.5°C.

Thereafter, the graft material powder having the bone morphogenetic protein adsorbed thereon and hydroxypropyl methylcellulose powder are mixed and stirred to form a gel. The viscous gel thus formed can improve the adhesion of the graft material powder. For example, the stirring may be performed using a mixer. As the graft material powder is stirred with the hydroxypropyl methylcellulose in powder form, a product with homogeneous quality can be obtained.

According to one embodiment of the present disclosure, the volume ratio between the graft material powder having the bone morphogenetic protein adsorbed thereon and the hydroxypropyl methylcellulose powder may be 1:0.2 to 1:0.8. If the volume ratio of the hydroxypropyl methylcellulose powder is less than 0.2, it may be difficult to form a gel, and if the volume ratio of the hydroxypropyl methylcellulose powder is more than 0.8, it may be difficult to form an effective bone graft composition because the volume of the gel is larger than the volume of the graft material powder. In terms of the effects of the present disclosure, the volume ratio between the graft material powder having the bone morphogenetic protein adsorbed thereon and the hydroxypropyl methylcellulose powder may preferably be 1: 0.6 to 1: 0.7.

Thereafter, the mixture of the graft material powder and the hydroxypropyl methylcellulose powder, obtained by the mixing and stirring process, is freeze-dried under vacuum to form a sponge-like structure containing pores. A sponge-like structure containing a plurality of pores may also be formed by freeze-drying the mixture of the graft material powder and the hydroxypropyl methylcellulose powder, obtained by the mixing and stirring process, at a low temperature under vacuum.

A sponge-like structure including a porous structure may be formed by the freeze-drying treatment under vacuum. The gel may be absorbed into the graft material powder to form a sponge-like structure including a porous structure, and it is believed that the treatment under vacuum mainly contributes to the formation of the sponge-like structure including a porous structure.

According to one embodiment of the present disclosure, the method for preparing the bone graft composition may further include a packaging step.

According to one embodiment of the present disclosure, the method for preparing the bone graft composition may further include a step of placing the prepared bone graft composition including a sponge-like structure containing a plurality of pores in a snap tube sized to be inserted into a syringe. When the method further includes the step of placing the composition in a snap tube sized to be inserted into a syringe, the composition may be sized to be inserted into the syringe and thus may be inserted directly into the syringe without a separate process, so that the operation of the process for preparing the bone graft composition can be facilitated.

According to an embodiment of the present disclosure, the method for preparing the bone graft composition may further include a step of placing and sealing the bone graft composition including a sponge-like structure containing a plurality of pores, placed in the snap tube, in a syringe. When the bone graft composition is provided in the syringe, it is possible to ensure ease of use and significantly reduce the possibility of contamination that may occur during use.

According to one embodiment of the present disclosure, the method for preparing the bone graft composition may further include a step of sterilizing the composition.

In one embodiment of the present disclosure, the bone graft composition including a sponge-like structure containing a plurality of pores may be sterilized by ethylene oxide gas. For example, the concentration of the ethylene oxide gas may be 450 to 1,200 mg/l.

If the concentration of the ethylene oxide gas is less than 450 mg/l, sterilization may be insufficient, and if the concentration of the ethylene oxide gas is more than 1,200 mg/l, denaturation of the bone morphogenetic protein may occur.

According to one embodiment of the present disclosure, the bone graft composition including a sponge-like structure containing a plurality of pores may be sterilized by gamma-ray irradiation. For example, the dose of the gamma-ray irradiation may be 10 to 25 kGy. If the dose of the gamma-ray irradiation is less than 10 kGy, sterilization may be insufficient, and if the dose of the gamma-ray irradiation is more than 25 kGy, denaturation of the bone morphogenetic protein may occur.

The bone graft composition prepared according to the above-described method has a certain dissolution rate under certain conditions for application of the composition to the human body, for example, application of the composition to teeth. This dissolution rate may be determined by the content of hydroxypropyl methylcellulose (HPMC) or the like in the bone graft composition.

For example, in the case in which the bone graft composition is applied to teeth, when a dental operator applies the bone graft composition to a missing tooth, the hydroxypropyl methylcellulose (HPMC) contained in the bone graft composition should reach a certain dissolution rate or more within a certain time so that the composition can be applied to the missing tooth with an appropriate adhesion and can fit the shape of the missing tooth. After the bone graft composition is applied to the missing tooth during the medical procedure, the phenomenon that the bone graft composition flows out around or detaches should not occur, and a phenomenon should also not occur in which the hydroxypropyl methylcellulose (HPMC) in the bond graft composition does not dissolve so that the bone graft composition cannot adhere to the bone defect portion, making it impossible to perform the medical procedure. Therefore, the bone graft composition requires critical dissolution conditions of hydroxypropyl methylcellulose (HPMC), and there may occur a difference between the functions thereof depending on the dissolution conditions of hydroxypropyl methylcellulose (HPMC).

When the bone graft composition is applied, the additive hydroxypropyl methylcellulose (HPMC) should reach a dissolution rate of 50% or more within 48 hours so that the composition may effectively function as a bone graft material. If the hydroxypropyl methylcellulose (HPMC) does not reach a dissolution rate of 50% or more within 48 hours, the phenomenon that undissolved hydroxypropyl methylcellulose (HPMC) is cured will occur, and as the curing rate thereof increases, a space in the bone graft composition, into which blood can flow, decreases, so that the composition may not function as a bone graft material.

Meanwhile, if the dissolution rate of the hydroxypropyl methylcellulose (HPMC) reaches 89% or more (a residual rate of less than 11%) within 48 hours, the bone graft composition cannot maintain its shape because the content of the HPMC in the bone graft composition is low. In addition, in this case, the composition cannot agglomerate, and hence it is virtually impossible to perform the medical procedure. Furthermore, in this case, even if the medical procedure is completed, the adhesion of the bone graft material may be reduced by physical activities of the person who received the medical procedure, such as salivary gland activity, mastication motion by eating, breathing, conversation, etc., and side effects may occur, such as detachment of the shaped material implanted to fit the defect shape.

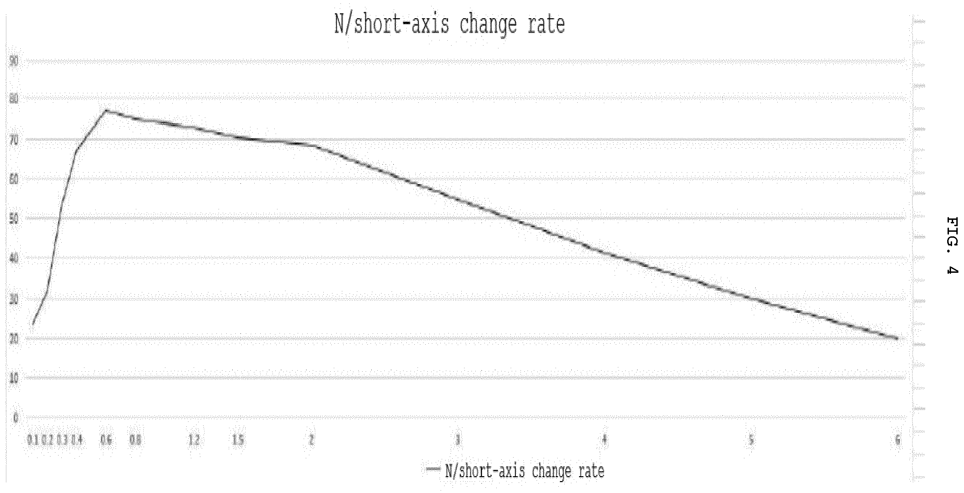

In order to ensure shape retainability, the bone graft composition according to one embodiment of the present disclosure may contain hydroxypropyl methylcellulose in an amount of 0.3 to 3 parts by weight, more preferably 0.4 to 2 parts by weight, based on 1 part by weight of the porous bone graft material. In this case, the shape retentability of the composition is further enhanced. As can be seen from the Experimental Example to be described later, it was confirmed that, as the content of the hydroxypropyl methylcellulose increased, the short-axis change rate increased, and the maximum breaking force tended to increase and then decrease. As shown in FIG. 4, it can be seen that this increase or decrease was rapid. Through this, it was confirmed that a bone graft composition having shape retainability can be provided when the content of the hydroxypropyl methylcellulose is 0.3 to 3 parts by weight based on 1 part by weight of the bone graft material. If the content of the hydroxypropyl methylcellulose is less than 0.3 parts by weight based on 1 part by weight of the bone graft material, the effect of adding the hydroxypropyl methylcellulose (HPMC) may be insignificant due to an excessively low content of the hydroxypropyl methylcellulose (HPMC), and thus the bone graft material can be easily pressed and broken even by low pressure (force) so that the shape of the bone graft material cannot be suitably retained. On the other hand, if the content of the hydroxypropyl methylcellulose is more than 3 parts by weight based on 1 part by weight of the bone graft material, the porous bone graft material can be easily pressed even by a small force due to an excessively high content of the hydroxypropyl methylcellulose (HPMC) and even the short-axis change rate can increase so that the shape of the bone graft material cannot be suitably retained.

A bone graft composition kit according to another embodiment of the present disclosure includes the above-described bone graft composition and a syringe containing the composition. By providing the syringe containing the bone graft composition, it is possible to ensure ease of use and significantly reduce the possibility of contamination that may occur during use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-described embodiments.

A method for preparing a bone graft composition according to still another embodiment of the present disclosure includes steps of: preparing a bone morphogenetic protein solution by adding a bone morphogenetic protein to a solvent or adding the bone morphogenetic protein to the solvent and dissolving the bone morphogenetic protein in the solvent;
mixing and stirring graft material powder having the bone morphogenetic protein adsorbed thereon and hydroxypropyl methylcellulose powder to form a viscous gel that imparts shape retainability to the bone graft composition; and
forming a sponge-like structure containing a plurality of pores by freeze-drying the mixture of the graft material powder and the hydroxypropyl methylcellulose powder, obtained by the mixing and stirring process, at low temperature under vacuum. The bone graft composition prepared through these steps may have excellent effects in terms of activation of bone formation, biocompatibility, and ease of use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-described embodiments.

FIG. 1 is a flow diagram schematically showing a method for preparing a bone graft composition according to one embodiment of the present disclosure.

FIG. 4 shows "maximum breaking force (N) of bone graft composition sphere/short-axis change rate of bone graft composition sphere" as a function of the content (parts by weight) of hydroxypropyl methylcellulose, and shows the content (parts by weight) of hydroxypropyl methylcellulose for a bone graft composition having excellent shape retainability.

First, a bone morphogenetic protein solution is prepared by dissolving a bone morphogenetic protein in a solvent. The bone morphogenetic protein solution may be prepared by adding the bone morphogenetic protein to the solvent, or adding the bone morphogenetic protein to the solvent and dissolving the bone morphogenetic protein in the solvent.

The bone morphogenetic protein may be at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto. Preferably, the bone morphogenetic protein may be rhBMP-2 in terms of the bone formation effect of the present disclosure.

According to one embodiment of the present disclosure, the concentration of the bone morphogenetic protein in the bone morphogenetic protein solution may be 0.05 to 0.15 mg/ml, preferably 0.08 to 0.12 mg/ml. When the concentration of the bone morphogenetic protein is within the above-described range, bone formation by the bone morphogenetic protein may be activated. If the concentration of the bone morphogenetic protein is less than 0.05 mg/ml, the ability to form new bone may be reduced, and if the concentration of the bone morphogenetic protein is more than 0.15 mg/ml, it may cause adverse effects.

In addition, according to one embodiment of the present disclosure, the pH of the bone morphogenetic protein solution may be, for example, 4.6 to 5. When the pH is within the above-described range, bone formation by the bone morphogenetic protein may be activated. If the pH of the bone morphogenetic protein solution is less than 4.6, the ability to form new bone may be reduced, and if the pH of the bone morphogenetic protein solution is more than 5, the ability to form new bone may be reduced. For example, the pH may be adjusted using phosphate buffer saline. When the pH is adjusted using phosphate buffer saline, the bone morphogenetic protein may have the effect of forming new bone.

Thereafter, the bone morphogenetic protein is adsorbed onto graft material powder by soaking the graft material powder with the bone morphogenetic protein solution. The previously prepared graft material powder may be soaked with the bone morphogenetic protein solution by flushing the graft material powder with the bone morphogenetic protein solution or dropping the graft material powder into the bone morphogenetic protein solution, whereby the bone morphogenetic protein may be adsorbed onto the graft material powder.

The graft material powder may be autogenous bone, allogeneic bone, or xenogenic bone. For example, the graft material powder may be prepared by placing it in a snap tube.

The average particle diameter (D50) of the graft material powder may be 200 to 5,000 µm, preferably 250 to 1,000 µm. If the average particle diameter of the powder is less than 200 µm, the graft material may be absorbed rapidly, and thus osteoconduction required for bone formation may be insufficient, and if the average particle diameter of the powder is more than 5,000 µm, precise processing for application to a patient may be difficult.

According to one embodiment of the present disclosure, the step of adsorbing the bone morphogenetic protein onto the graft material powder may include a step of adsorbing the bone morphogenetic protein using a refrigerated centrifuge.

In some cases, the bone morphogenetic protein may also be suspended in the solution. However, when the bone morphogenetic protein is adsorbed while it is rotated at high speed using a centrifuge, the bone morphogenetic protein can be prevented from being suspended in the solution, and thus the bone morphogenetic protein may be easily adsorbed onto the surface or into the pores of the graft material powder. Only when the bone morphogenetic protein is adsorbed while it is rotated at high speed, it can be prevented from being suspended again after detachment from the graft material powder. If the bone morphogenetic protein is rotated at low speed, it can be suspended, and hence cannot be easily adsorbed. Under high-speed rotation, the bone morphogenetic protein can be adsorbed quickly onto the surface or into the pores of the graft material powder.

According to one embodiment of the present disclosure, the rotational speed of the refrigerated centrifuge may be 4,000 rpm or more. When the bone morphogenetic protein is adsorbed using the centrifuge, the higher the rotational speed, the better the adsorption. For example, the rotational speed of the centrifuge may be 4,000 rpm or more, and when this rotational speed range is satisfied, the bone morphogenetic protein can be prevented from being suspended in the solution.

According to one embodiment of the present disclosure, the step of adsorbing the bone morphogenetic protein using the refrigerated centrifuge may be performed at a cold temperature of 5°C or below. As the step of adsorbing the bone morphogenetic protein using the refrigerated centrifuge is performed at a cold temperature of 5°C or below, it is possible to maximize the effect of adsorbing the bone morphogenetic protein onto the surface or into the pores of the graft material powder through rotation while preventing the denaturation of the bone morphogenetic protein that is weak to heat, by preventing the temperature of the solution from being increased due to rotation. The cold temperature may be a temperature at which the solution does not freeze. For example, the cold temperature may be 5°C or below, preferably 0.5 to 1.5°C.

Thereafter, the graft material powder having the bone morphogenetic protein adsorbed thereon and hydroxypropyl methylcellulose powder are mixed and stirred to form a gel. The viscous gel thus formed can improve the adhesion of the graft material powder. For example, the stirring may be performed using a mixer. As the graft material powder is stirred with the hydroxypropyl methylcellulose in powder form, a product with homogeneous quality can be obtained.

According to one embodiment of the present disclosure, the volume ratio between the graft material powder having the bone morphogenetic protein adsorbed thereon and the hydroxypropyl methylcellulose powder may be 1:0.2 to 1:0.6. If the volume ratio of the hydroxypropyl methylcellulose powder is less than 0.2, it may be difficult to form a gel, and if the volume ratio of the hydroxypropyl methylcellulose powder is more than 0.6, it may be difficult to form an effective bone graft composition because the volume of the gel is larger than the volume of the graft material powder. In terms of the effects of the present disclosure, the volume ratio between the graft material powder having the bone morphogenetic protein adsorbed thereon and the hydroxypropyl methylcellulose powder may preferably be 1:0.25 to 1: 0.35.

Thereafter, the mixture of the graft material powder and the hydroxypropyl methylcellulose powder, obtained by the mixing and stirring process, is freeze-dried under vacuum to form a sponge-like structure containing pores. A sponge-like structure containing a plurality of pores may also be formed by freeze-drying the mixture of the graft material powder and the hydroxypropyl methylcellulose powder, obtained by the mixing and stirring process, at a low temperature under vacuum.

A sponge-like structure including a porous structure may be formed by the freeze-drying treatment under vacuum. The gel may be absorbed into the graft material powder to form a sponge-like structure including a porous structure, and it is believed that the treatment under vacuum mainly contributes to the formation of the sponge-like structure including a porous structure.

According to one embodiment of the present disclosure, the method for preparing the bone graft composition may further include a packaging step.

According to one embodiment of the present disclosure, the method for preparing the bone graft composition may further include a step of placing the prepared bone graft composition including a sponge-like structure containing a plurality of pores in a snap tube sized to be inserted into a syringe. When the method further includes the step of placing the composition in a snap tube sized to be inserted into a syringe, the composition may be sized to be inserted into the syringe and thus may be inserted directly into the syringe without a separate process, so that the operation of the process for preparing the bone graft composition can be facilitated.

According to an embodiment of the present disclosure, the method for preparing the bone graft composition may further include a step of placing and sealing the bone graft composition including a sponge-like structure containing a plurality of pores, placed in the snap tube, in a syringe. When the bone graft composition is provided in the syringe, it is possible to ensure ease of use and significantly reduce the possibility of contamination that may occur during use.

According to one embodiment of the present disclosure, the method for preparing the bone graft composition may further include a step of sterilizing the composition.

In one embodiment t of the present disclosure, the bone graft composition including a sponge-like structure containing a plurality of pores may be sterilized by ethylene oxide gas. For example, the concentration of the ethylene oxide gas may be 450 to 1,200 mg/l.

If the concentration of the ethylene oxide gas is less than 450 mg/l, sterilization may be insufficient, and if the concentration of the ethylene oxide gas is more than 1,200 mg/l, denaturation of the bone morphogenetic protein may occur.

According to one embodiment of the present disclosure, the bone graft composition including a sponge-like structure containing a plurality of pores may be sterilized by gamma-ray irradiation. For example, the dose of the gamma-ray irradiation may be 10 to 25 kGy. If the dose of the gamma-ray irradiation is less than 10 kGy, sterilization may be insufficient, and if the dose of the gamma-ray irradiation is more than 25 kGy, denaturation of the bone morphogenetic protein may occur.

The bone graft composition according to the embodiment of the present disclosure, which may be prepared according to the above-described method, may have shape retainability for application of the composition to the human body, for example, application of the bone graft composition to missing teeth. This shape retainability may be determined by the content of hydroxypropyl methylcellulose (HPMC) or the like in the bone graft composition.

For example, in the case in which a bone graft material is applied to teeth, when a dental operator applies the bone graft material to a missing tooth, the bone graft material should have a predetermined degree of plasticity so that it can be deformed to fit the shape of the missing tooth. In addition, after the bone graft material is applied to the missing tooth, the phenomenon that the bone graft composition flows out around or detaches should not occur. Thus, the bone graft material should have a predetermined degree or more of plasticity, and should also have a predetermined degree or more of strength that can resist gravity or the movement of the bone (tooth) defect.

This degree of strength and plasticity may be defined as shape retainability. Shape retainability can be defined by the change rate of the short-axis that can be quantified as the spherical shape of a spherical sample having a diameter of XXX mm is deformed into an elliptical shape when a force of XXX N is applied to the sample, in order to quantify the objective degree of shape retainability.

In order to satisfy the above-described plasticity and strength, the shape retainability of the bone graft composition should be 50 or more. If the shape retainability is less than 50, the bone graft composition may be difficult to apply to a bone defect, due to the strong elasticity thereof. That is, when a medical operator applies the bone graft composition to a bone defect, the bone graft composition should be deformed to fit the shape of the bone defect, but if the elasticity of the bone graft composition is strong, the bone graft composition may be difficult to deform due to the high resilience of the bone graft composition. On the other hand, if the shape retainability is less than 50, the bone graft composition may flow out around during a medical procedure such as hydration that is performed by a medical operator, and hence it is virtually impossible to perform the medical procedure.

Thus, in order to ensure the applicability of the bone graft composition while ensuring the ease of the actual medical procedure, the "maximum breaking force (N)/short-axis change rate" of the bone graft composition should be 50 or more, and in this case, the bone graft composition is easy to deform to fit the shape of the bone defect due to the excellent shape retainability thereof, and it is possible to obtain a bone graft composition that do not have problems, such as a phenomenon in which the bone graft material flows out around during a medical procedure such as hydration which is performed by a medical operator. To this end, the content of hydroxypropyl methylcellulose is 0.3 to 3 parts by weight based on 1 part by weight of the bone graft material, and in this case, it is possible to obtain a bone graft composition having shape retainability of 50 or more.

According to one embodiment of the present disclosure, a bone graft composition showing optimal bone graft properties during a medical procedure may contain hydroxypropyl methylcellulose in an amount of 0.15 to 6 parts by weight, more preferably 0.3 to 3 parts by weight, based on 1 part by weight of the bone graft material. If the content of the hydroxypropyl methylcellulose is less than 0.3 parts by weight based on 1 part by weight of the bone graft material, the composition has insufficient adhesion to a bone defect, and hence is highly likely to detach from the bone defect during use, and the hydroxypropyl methylcellulose has little or no effect on the osmotic properties of the bone graft composition because the content thereof is insignificant. If the content of the hydroxypropyl methylcellulose is more than 3 parts by weight based on 1 part by weight of the bone graft material, the hydroxypropyl methylcellulose may interfere with bone formation by interfering with the wettability, hygroscopicity or the like of xenogenic bone, and hydroxypropyl methylcellulose may not easily dissolve due to curing thereof, and may have a great effect on the osmotic pressure of the composition due to flowing out thereof, and thus the composition may not suitably function as a bone graft material.

The bone graft composition containing the hydroxypropyl methylcellulose is used as a solution obtained by dissolving it in a solvent. As the solvent, any solvent that may be used in the art may be suitably selected and used, and for example, water is used. Since the physical properties of the bone graft composition, such as dissolution rate, concentration, osmotic properties, and shape retainability, vary depending on the conditions of the solvent, it is important to select suitable solvent conditions for the practice of the present disclosure.

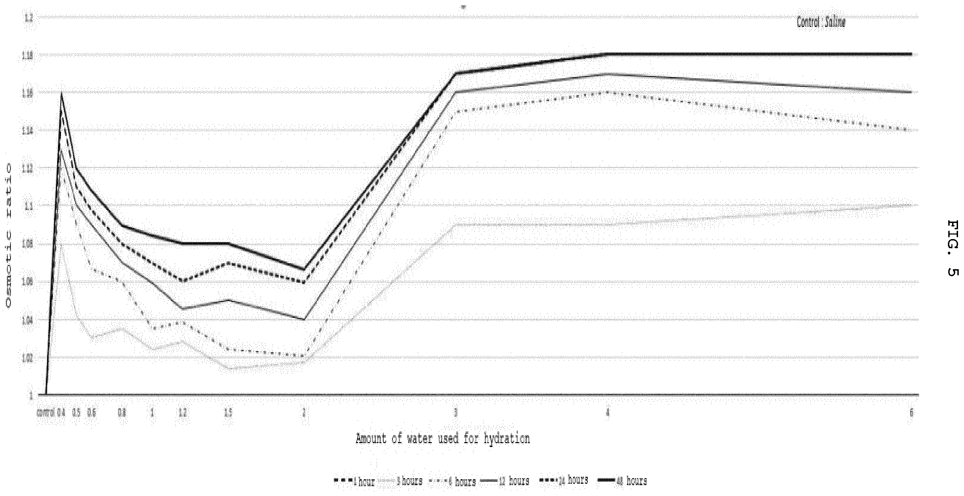

According to one embodiment of the present disclosure, the bone graft solution may be prepared by mixing 1 part by weight of the bone graft composition containing hydroxypropyl methylcellulose with 0.5 to 2 parts by weight of the solvent (water), in order for the bone graft solution to show optimal properties during a medical procedure, specifically in order for the bone graft solution to form an optimum osmotic pressure with another solution. As can be seen from the Experimental Example to be described later, the content of the solvent should be within a certain range so that the bone graft solution can form an appropriate osmotic phenomenon with another solution. As shown in FIG. 5, it can be seen that 0.5 to 2 parts by weight of the solvent (e.g., water) should be mixed with 1 part by weight of the bone graft composition containing hydroxypropyl methylcellulose, in order to form an appropriate osmotic phenomenon.

If the content of the solvent (water) is less than 0.5 parts by weight based on 1 part by weight of the bone graft composition containing hydroxypropyl methylcellulose, dissolution may not easily occur because the amount of the solvent (water) is excessively small, and hence the hydroxypropyl methylcellulose may not easily bond with the bone graft material and may not sufficiently exhibit its function. Generally, the composition may be so stiff that it cannot be suitable for bone formation. In addition, it may form a strong osmotic pressure with another solution, and thus the bone graft material may not retain its shape and the function thereof can be impaired.

If the content of the solvent (water) is more than 2 parts by weight based on 1 part by weight of the bone graft composition containing hydroxypropyl methylcellulose, the amount of the solvent (water) is so large that the solution has a property of flowing without having proper viscosity, and thus the composition may not have shape retainability. In addition, dissolution of the hydroxypropyl methylcellulose may proceed rapidly due to a high content of the solvent (water), and thus the hydroxypropyl methylcellulose may flow out so that it cannot exhibit its function. In addition, it may form a strong osmotic pressure with another solution, and thus the bone graft material may not retain its shape and the function thereof can be impaired.

Thus, as a solvent condition for allowing the bone graft composition containing hydroxypropyl methylcellulose to sufficiently exhibit the function of the hydroxypropyl methylcellulose and to retain the shape of the bone graft material, it is preferred that 0.5 to 2 parts by weight of the solvent (water) is mixed with 1 part by weight of the bone graft composition containing hydroxypropyl methylcellulose to prepare a bone graft solution.

A bone graft composition kit according to another embodiment of the present disclosure includes the above-described bone graft composition and a syringe containing the composition. By providing the syringe containing the bone graft composition, it is possible to ensure ease of use and significantly reduce the possibility of contamination that may occur during use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-descried embodiments.

A method for preparing a bone graft composition according to still another embodiment of the present disclosure includes steps of: preparing a bone morphogenetic protein solution by adding a bone morphogenetic protein to a solvent or adding the bone morphogenetic protein to the solvent to dissolve the bone morphogenetic protein in the solvent;
mixing and stirring graft material powder having the bone morphogenetic protein adsorbed thereon and hydroxypropyl methylcellulose powder to form a viscous gel; and
forming a sponge-like structure containing a plurality of pores by freeze-drying the mixture of the graft material powder and the hydroxypropyl methylcellulose powder, obtained by the mixing and stirring process, at low temperature under vacuum. The bone graft composition prepared through these steps may have excellent effects in terms of activation of bone formation, biocompatibility, and ease of use.

However, in the description of this embodiment, the description of a portion that overlaps with that of the above-described embodiments is omitted for a clearer and more concise explanation. Even though the description of that portion is omitted, the portion is not excluded from the present disclosure and the scope of rights thereof should be admitted in the same manner as that of the above-described embodiments.

FIG. 1 is a flow diagram schematically showing a method for preparing a bone graft composition according to one embodiment of the present disclosure.

FIG. 5 shows result data for an Experimental Example of the present disclosure, obtained by mixing saline with bone graft solutions formed using varying amounts (parts by weight) of a solvent, and expressing the time-dependent change in osmotic pressure of the mixed saline compared to that of pure saline as osmotic ratio.

First, a bone morphogenetic protein solution is prepared by dissolving a bone morphogenetic protein in a solvent. The bone morphogenetic protein solution may be prepared by adding the bone morphogenetic protein to the solvent, or adding the bone morphogenetic protein to the solvent and dissolving the bone morphogenetic protein in the solvent.

The bone morphogenetic protein may be at least one selected from the group consisting of BMP-2, BMP-3, BMP-3b, BMP-4, BMP-5, BMP-6, BMP-7, BMP-8, BMP-9, BMP-10, BMP-11, BMP-12, BMP-13, BMP-14, BMP-15, BMP-16, BMP-17, BMP-18, recombinant bone morphogenetic proteins thereof, and bone morphogenetic proteins equivalent thereto. Preferably, the bone morphogenetic protein may be rhBMP-2 in terms of the bone formation effect of the present disclosure.

According to one embodiment of the present disclosure, the concentration of the bone morphogenetic protein in the bone morphogenetic protein solution may be 0.05 to 0.15 mg/ml, preferably 0.08 to 0.12 mg/ml. When the concentration of the bone morphogenetic protein is within the above-described range, bone formation by the bone morphogenetic protein may be activated. If the concentration of the bone morphogenetic protein is less than 0.05 mg/ml, the ability to form new bone may be reduced, and if the concentration of the bone morphogenetic protein is more than 0.15 mg/ml, it may cause adverse effects.

In addition, according to one embodiment of the present disclosure, the pH of the bone morphogenetic protein solution may be, for example, 4.6 to 5. When the pH is within the above-described range, bone formation by the bone morphogenetic protein may be activated. If the pH of the bone morphogenetic protein solution is less than 4.6, the ability to form new bone may be reduced, and if the pH of the bone morphogenetic protein solution is more than 5, the ability to form new bone may be reduced. For example, the pH may be adjusted using phosphate buffer saline. When the pH is adjusted using phosphate buffer saline, the effect of forming new bone may be obtained.

Thereafter, the bone morphogenetic protein is adsorbed onto graft material powder by soaking the graft material powder with the bone morphogenetic protein solution. The previously prepared graft material powder may be soaked with the bone morphogenetic protein solution by flushing the graft material powder with the bone morphogenetic protein solution or dropping the graft material powder into the bone morphogenetic protein solution, whereby the bone morphogenetic protein may be adsorbed onto the graft material powder.

The graft material powder may be autogenous bone, allogeneic bone, or xenogenic bone. For example, the graft material powder may be prepared by placing it in a snap tube.

The average particle diameter (D50) of the graft material powder may be 200 to 5,000 µm, preferably 250 to 1,000 µm. If the average particle diameter of the powder is less than 200 µm, the graft material may be absorbed rapidly, and thus osteoconduction required for bone formation may be insufficient, and if the average particle diameter of the powder is more than 5,000 µm, precise processing of the graft material powder during application to a patient may be difficult.

According to one embodiment of the present disclosure, the step of adsorbing the bone morphogenetic protein onto the graft material powder may include a step of adsorbing the bone morphogenetic protein using a refrigerated centrifuge.

In some cases, the bone morphogenetic protein may also be suspended in the solution. However, when the bone morphogenetic protein is adsorbed while it is rotated at high speed using a centrifuge, the bone morphogenetic protein can be prevented from being suspended in the solution, and thus the bone morphogenetic protein may be easily adsorbed onto the surface or into the pores of the graft material powder. Only when the bone morphogenetic protein is adsorbed while it is rotated at high speed, it can be prevented from being suspended again after detachment from the graft material powder. If the bone morphogenetic protein is rotated at low speed, it can be suspended, and hence cannot be easily adsorbed. Under high-speed rotation, the bone morphogenetic protein can be adsorbed quickly onto the surface or into the pores of the graft material powder.

According to one embodiment of the present disclosure, the rotational speed of the refrigerated centrifuge may be 4,000 rpm or more. When the bone morphogenetic protein is adsorbed using the centrifuge, the higher the rotational speed, the better the adsorption. For example, the rotational speed of the centrifuge may be 4,000 rpm or more, and when this rotational speed range is satisfied, the bone morphogenetic protein can be prevented from being suspended in the solution.

According to one embodiment of the present disclosure, the step of adsorbing the bone morphogenetic protein using the refrigerated centrifuge may be performed at a cold temperature of 5°C or below. As the step of adsorbing the bone morphogenetic protein using the refrigerated centrifuge is performed at a cold temperature of 5°C or below, it is possible to maximize the effect of adsorbing the bone morphogenetic protein onto the surface or into the pores of the graft material powder through rotation while preventing the denaturation of the bone morphogenetic protein that is weak to heat, by preventing the temperature of the solution from being increased due to rotation. The cold temperature may be a temperature at which the solution does not freeze. For example, the cold temperature may be 5°C or below, preferably 0.5 to 1.5°C.

Thereafter, the graft material powder having the bone morphogenetic protein adsorbed thereon and hydroxypropyl methylcellulose powder are mixed and stirred to form a gel. The viscous gel thus formed can improve the adhesion of the graft material powder. For example, the stirring may be performed using a mixer. As the graft material powder is stirred with the hydroxypropyl methylcellulose in powder form, a product with homogeneous quality can be obtained.

According to one embodiment of the present disclosure, the volume ratio between the graft material powder having the bone morphogenetic protein adsorbed thereon and the hydroxypropyl methylcellulose powder may be 1:0.2 to 1:0.6. If the volume ratio of the hydroxypropyl methylcellulose powder is less than 0.2, it may be difficult to form a gel, and if the volume ratio of the hydroxypropyl methylcellulose powder is more than 0.6, it may be difficult to form an effective bone graft composition because the volume of the gel is larger than the volume of the graft material powder. In terms of the effects of the present disclosure, the volume ratio between the graft material powder having the bone morphogenetic protein adsorbed thereon and the hydroxypropyl methylcellulose powder may preferably be 1: 0.25 to 1: 0.35.

Thereafter, the mixture of the graft material powder and the hydroxypropyl methylcellulose powder, obtained by the mixing and stirring process, is freeze-dried under vacuum to form a sponge-like structure containing pores. A sponge-like structure containing a plurality of pores may also be formed by freeze-drying the mixture of the graft material powder and the hydroxypropyl methylcellulose powder, obtained by the mixing and stirring process, at a low temperature under vacuum.

A sponge-like structure including a porous structure may be formed by the freeze-drying treatment under vacuum. The gel may be absorbed into the graft material powder to form a sponge-like structure including a porous structure, and it is believed that the treatment under vacuum mainly contributes to the formation of the sponge-like structure including a porous structure.

According to one embodiment of the present disclosure, the method for preparing the bone graft composition may further include a packaging step.

According to one embodiment of the present disclosure, the method for preparing the bone graft composition may further include a step of placing the prepared bone graft composition containing a sponge-like structure containing a plurality of pores in a snap tube sized to be inserted into a syringe. When the method further includes the step of placing the composition in a snap tube sized to be inserted into a syringe, the composition may be sized to be inserted into the syringe and thus may be inserted directly into the syringe without a separate process, so that the operation of the process for preparing the bone graft composition can be facilitated.

According to an embodiment of the present disclosure, the method for preparing the bone graft composition may further include a step of placing and sealing the bone graft composition containing a sponge-like structure containing a plurality of pores, placed in the snap tube, in a syringe. When the bone graft composition is provided in the syringe, it is possible to ensure ease of use and significantly reduce the possibility of contamination that may occur during use.

According to one embodiment of the present disclosure, the method for preparing the bone graft composition may further include a step of sterilizing the composition.

In one embodiment of the present disclosure, the bone graft composition including a sponge-like structure containing a plurality of pores may be sterilized by ethylene oxide gas. For example, the concentration of the ethylene oxide gas may be 450 to 1,200 mg/l.

If the concentration of the ethylene oxide gas is less than 450 mg/l, sterilization may be insufficient, and if the concentration of the ethylene oxide gas is more than 1,200 mg/l, denaturation of the bone morphogenetic protein may occur.

According to one embodiment of the present disclosure, the bone graft composition including a sponge-like structure containing a plurality of pores may be sterilized by gamma-ray irradiation. For example, the dose of the gamma-ray irradiation may be 10 to 25 kGy. If the dose of the gamma-ray irradiation is less than 10 kGy, sterilization may be insufficient, and if the dose of the gamma-ray irradiation is more than 25 kGy, denaturation of the bone morphogenetic protein may occur.

The bone graft composition prepared according to the above-described method should have an ensured hydration ability for application of the composition to the human body, for example, application of the composition to teeth. In other words, the bone graft composition should have critical osmotic properties. Allowing the bone graft composition to form a certain osmotic ratio with another solution (e.g., saline) is an important factor in the ability of the bone graft material to be maintained at a constant concentration to thereby retain its shape and function. This property may be determined depending on the content of HPMC or the like contained in the bone graft composition, as well as the condition of the solvent for forming the solution.

For example, in the case in which the bone graft composition is applied to teeth, a dental operator applies the bone graft composition, which is provided as powder or the like, to a missing tooth after hydrating the composition. If the HPMC contained in the bone graft composition flows out in large amounts (that is, the osmotic pressure of the hydrated composition increases) during this hydration process, the HPMC cannot remain inside the bone graft material, and thus the bone graft material cannot retain its shape and also cannot agglomerate, making it impossible to perform the medical procedure. For this reason, the bone graft composition should have an osmotic properties within a predetermined range so that it can retain its shape without being influenced by external conditions. Furthermore, if an external environment such as water or saliva is formed after the bone graft composition is applied to the missing tooth, a phenomenon may occur in which the bone graft composition flows out around or detaches, and thus a problem may arise in that an external substance flows into the missing tooth. For this reason, the bone graft composition should have an osmotic properties within a predetermined range, and furthermore, have not only internal physical properties, but also properties that resist external conditions.

Actually, when the osmotic pressure of saline is set to 100% as a reference value, the saline should reach an osmotic pressure of 104 to 112% within 12 to 48 hours after addition to the bone graft solution. If the osmotic pressure is more than 112%, the HPMC may flow out during the hydration process, and thus the shape retainability of the bone graft composition and the adhesion thereof to a bone defect may significantly decrease, making it impossible to apply the bone graft composition. On the other hand, if the osmotic pressure is less than 104%, the bone graft composition may not be easily hydrated, and thus may be difficult to deform plastically to the bone defect, thus adversely affecting bone regeneration.

Hereinafter, preferred examples will be presented to help the understanding of the present disclosure. However, these examples are merely to illustrate the present disclosure and are not intended to limit the scope of the present disclosure as defined in the appended claims. In addition, it will be obvious to those skilled in the art that various changes and modifications of these examples are possible without departing from the scope and technical spirit of the present disclosure. In addition, it is to be understood that these changes and modifications also fall within the appended claims.

### Experimental Example 1

### 1. Experiment for Examining Residual Amount and Solubility of Hydroxypropyl Methylcellulose (HPMC) Depending on the Content Thereof

As shown in Table 1 below, various amounts (0.1 to 6 parts by weight) of HPMC were each added to 0.25 g of a bone graft material. Each of the bone graft material/HPMC mixtures was dissolved in a solvent (water), and the residual amount of the HPMC was examined over time. The amount of HPMC initially added was set to 100% as a reference value, and the residual amount of the HPMC remaining after dissolution was expressed as a percentage (%) relative to the reference value (100%). It can be interpreted that the percentage of the residual amount, which is closer to 100%, indicates that little or no dissolution occurred, and the percentage of the residual amount, which is closer to 0%, indicates that dissolution of most of the HPMC occurred. In other words, it can be interpreted that when the percentage of the residual amount is 100%, the dissolution rate is 0%, and when the percentage of the residual amount is 0%, the dissolution rate is 100%.

The amount of the solvent (water) corresponds to an optimal degree of hydration to which the mixture can dissolve well. The amount of the solvent is 1 to 1.5 times, for example, 1.2 times, the total weight of the mixture.

**Table 1**

| | 0 hr | 1 hr | 3 hr | 6 hr | 12 hr | 24 hr | 48 hr |
|---|---|---|---|---|---|---|---|
| 0.1 | 100% | 0% | 0% | 0% | 0% | 0% | 0% |
| 0.2 | 100% | 15% | 3% | 0% | 0% | 0% | 0% |
| 0.3 | 100% | 62% | 55% | 50% | 39% | 29% | 11% |
| 0.3 | 100% | 64% | 60% | 54% | 43% | 31% | 14% |
| 0.6 | 100% | 80% | 70% | 58% | 48% | 33% | 20% |
| 0.8 | 100% | 85% | 78% | 71% | 58% | 44% | 22% |
| 1.2 | 100% | 93% | 88% | 82% | 67% | 57% | 30% |
| 1.5 | 100% | 94% | 89% | 87% | 73% | 68% | 35% |
| 2 | 100% | 95% | 90% | 88% | 75% | 73% | 42% |
| 3 | 100% | 99% | 94% | 92% | 81% | 75% | 50% |
| 4 | 100% | 99% | 96% | 93% | 85% | 78% | 60% |
| 5 | 100% | 100% | 98% | 95% | 88% | 81% | 70% |
| 6 | 100% | 100% | 100% | 100% | 99% | 99% | 98% |

As shown in Table 1 above, it can be seen that, as the dissolution time increased, the residual amount decreased and the dissolution rate increased. In addition, it can be seen that, as the amount of hydroxypropyl methylcellulose (HPMC) increased, the residual amount at the same time point increased and the dissolution rate decreased.

FIG. 2 shows the time-dependent residual rate of hydroxypropyl methylcellulose as a function of the content (parts by weight) thereof, calculated based on the result data (Table 1) obtained in Experimental Example 1. In the graph of FIG. 2, a value on the Y-axis, which is closer to 1, indicates a residual rate of 100% (a dissolution rate of 0%), and a value on the Y-axis, which is closer to 0, indicates a residual rate of 0% (a dissolution rate of 100%) .

The dissolution rate of the hydroxypropyl methylcellulose in the bone graft composition may require different conditions depending on the application for use of the composition, an environment in which the composition is used, and the purpose of use of the composition. The content ratio between the porous bone graft material and the hydroxypropyl methylcellulose may be determined in consideration of the intended use of the composition and the dissolution time and dissolution rate of the hydroxypropyl methylcellulose.

Under a condition in which 50% or more of hydroxypropyl methylcellulose needs to be dissolved within 48 hours, 0.1 to 3 parts by weight of the hydroxypropyl methylcellulose may be mixed with 1 part by weight of the porous bone graft material to form a bone graft composition. In another example, under a condition in which 60% or more of hydroxypropyl methylcellulose needs to be dissolved within 48 hours, 0.1 to 2 parts by weight of the hydroxypropyl methylcellulose may be mixed with 1 part by weight of the porous bone graft material to form a bone graft composition. In still another example, under a condition in which 70% or more of hydroxypropyl methylcellulose needs to be dissolved within 48 hours, 0.1 to 1.2 parts by weight of the hydroxypropyl methylcellulose may be mixed with 1 part by weight of the porous bone graft material to form a bone graft composition. In yet another example, under a condition in which 80% or more of hydroxypropyl methylcellulose needs to be dissolved within 48 hours, 0.1 to 0.6 parts by weight of the hydroxypropyl methylcellulose may be mixed with 1 part by weight of the porous bone graft material to form a bone graft composition. In consideration of the functional aspect of the bone graft material, 0.3 parts by weight or more of the hydroxypropyl methylcellulose is preferably mixed with 1 part by weight of the porous bone graft material.

In another example, under a condition in which 50% or more of hydroxypropyl methylcellulose needs to be dissolved within 24 hours, 0.1 to 1.0 part by weight of the hydroxypropyl methylcellulose may be mixed with 1 part by weight of the porous bone graft material to form a bone graft composition. In addition, in consideration of the functional aspect of the bone graft material, 0.3 to 1.0 part by weight of the hydroxypropyl methylcellulose may be mixed with 1 part by weight of the porous bone graft material to form a bone graft composition.

In another example, under a condition in which 50% or more of hydroxypropyl methylcellulose needs to be dissolved within 12 hours, 0.1 to 0.6 parts by weight of the hydroxypropyl methylcellulose may be mixed with 1 part by weight of the porous bone graft material to form a bone graft composition. In addition, in consideration of the functional aspect of the bone graft material, 0.3 to 0.6 parts by weight of the hydroxypropyl methylcellulose may be mixed with 1 part by weight of the porous bone graft material to form a bone graft composition.

### Experimental Example 2

### 2. Experiment for Examining Volume Reduction Rate and Solubility Depending on Content of Hydroxypropyl Methylcellulose (HPMC)

As shown in Table 2 below, various amounts (0.1 to 6 parts by weight) of HPMC were each added to 0.25 g of a bone graft material. Each of the bone graft material/HPMC mixtures was dissolved (hydrated) in a solvent (water), and the residual amount of the HPMC was examined over time. Specifically, the hydrated sample is placed closely in a 15-ml conical tube, and then the initial volume thereof was measured, and then the portion not occupied by the sample was cut from the conical tube. Next, the opening of the conical tube remaining after cutting was covered with a mesh so that only the dissolved portion of the sample could through the opening.

Thereafter, the conical tube was placed in an ultrasonic cleaner maintained at the human body temperature (for example, 37°C), and purified water was circulated at a constant flow rate, and after 48 hours, the volume of the sample was measured. Before measurement of the volume of the sample, purified water remaining in the sample may be removed.

Depending on the amount of HMPC added, the volume reduction rate in the environment similar to that in the human body was measured. It can be interpreted that a higher volume reduction rate indicates a higher dissolution rate of the sample, and a lower volume reduction rate indicates a lower solubility of the sample.

The amount of the solvent (water) corresponds to an optimal degree of hydration to which the mixture can dissolve well. The amount of the solvent is 1 to 1.5 times, for example, 1.2 times, the total weight of the mixture.

**Table 2**

| Parts by weight of HPMC | Initial volume (cc) | Volume (cc) after 48 hours | Volume reduction rate (%) |
|---|---|---|---|
| 0.1 | 0.270 | 0.101 | 62.58% |
| 0.2 | 0.529 | 0.307 | 42.01% |
| 0.3 | 0.652 | 0.544 | 16.54% |
| 0.4 | 0.766 | 0.683 | 10.82% |
| 0.6 | 1.131 | 1.010 | 10.70% |
| 0.8 | 1.275 | 1.136 | 10.92% |
| 1.2 | 1.987 | 1.791 | 9.85% |
| 1.5 | 2.312 | 2.090 | 9.61% |
| 2 | 2.768 | 2.544 | 7.73% |
| 3 | 4.080 | 3.816 | 6.46% |
| 4 | 4.378 | 4.728 | -7.99% |
| 5 | 5.426 | 5.954 | -9.72% |
| 6 | 5.672 | 6.322 | -1.46% |

**Table 3**

| Parts by weight of HPMC | Initial weight (g) | Weight (g) after 48 hours |
|---|---|---|
| 0.1 | 1.630 | 0.610 |
| 0.2 | 3.190 | 1.850 |
| 0.3 | 3.930 | 3.280 |
| 0.4 | 4.620 | 4.120 |
| 0.6 | 6.820 | 6.090 |
| 0.8 | 7.690 | 6.850 |
| 1.2 | 11.980 | 10.800 |
| 1.5 | 13.940 | 12.600 |
| 2 | 16.690 | 15.400 |
| 3 | 24.600 | 23.010 |
| 4 | 26.400 | 28.510 |
| 5 | 32.720 | 35.900 |
| 6 | 34.200 | 38.120 |

As shown in Tables 2 and 3 above, it can be seen that, depending on the content (parts by weight) of HPMC, the volume and weight of the sample changed after 48 hours in the human body environment. In addition, it can be seen that, as the content of hydroxypropyl methylcellulose (HPMC), the residual amount of the HPMC increased and the dissolution rate thereof decreased.

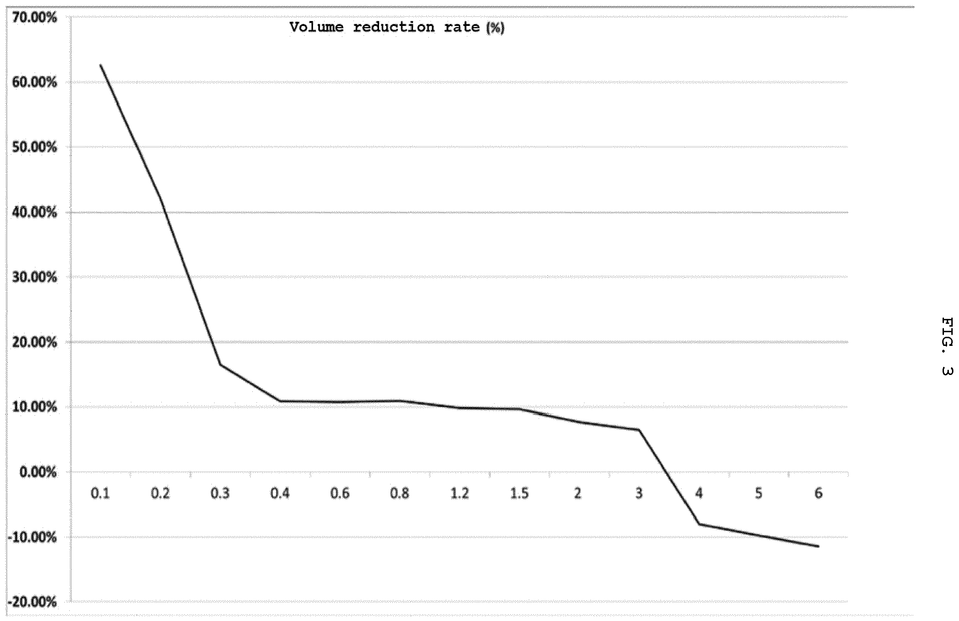

FIG. 3 shows volume reduction rates depending on the content (parts by weight) of hydroxypropyl methylcellulose (HPLC), calculated based on the result data (Table 2) obtained in Experimental Example 2 of the present disclosure.

As shown in FIG. 3, it can be seen that, when the content of the HPMC in the bone graft composition was 0.2 parts by weight, the volume reduction rate was 40%, and when the content of the HPMC was 0.3 parts by weight, the volume reduction rate decreased rapidly to 16.54%. In addition, it can be seen that, when the content of the HPMC was 0.2 parts by weight, the volume reduction rate was 40%, and when the content of the HPMC in the bone graft composition was 3 parts by weight, the volume reduction rate was decreased rapidly to 6.46%, but when the content of the HPMC was 4 parts by weight, the volume reduction rate has a negative value.

This means that, when the content of the HPMC is less than 0.3 parts by weight based on 1 part by weight of the bone graft material, the HPMC dissolves too quickly and flows out in the human body environment and flows out, and hence the volume of the sample is not maintained and the HPMC can neither promote nor help bone formation. On the other hand, if the content of the HPMC is more than 3 parts by weight based on 1 part by weight of the bone graft material, the volume of the HPMC becomes greater than the volume of the bone graft material, and thus a phenomenon will arise in which a shape in which the HPMC surrounds the bone graft material is formed, and thus the volume becomes larger while the HPMC absorbs external moisture. As can be seen in Table 3 above, this can be confirmed from the fact that when the content of the HPMC was more than 3 parts by weight based on 1 part by weight of the bone graft material, the weight of the sample after the experiment rather increased due to absorption of external moisture.

Accordingly, in order to achieve the functional aspect of the bone graft material, 0.3 to 3 parts by weight may be mixed with 1 part by weight of the porous bone graft material.

### Experimental Example 3

### 3. Experiment for Examining Shape Retainability Depending on Content of Hydroxypropyl Methylcellulose (HPMC)

As shown in Table 4 below, varying amounts (0.1 to 6 parts by weight) of HPMC was each mixed with 0.25 g of a bone graft material, and then dissolved in a solvent (DBS) to form viscous gels which were then shaped into spheres. The size of each initial sphere is shown as the long-axis length and the short-axis axis length in Table 4 below. A pressing force was applied to each of the spheres using a push-pull gage, and the short-axis length at the maximum breaking force (N) immediately before each sphere was broken, that is, the maximum peak value, was measured. The short-axis change rate obtained by comparing the short-axis length at the maximum breaking force with the initial short-axis length is shown in Table 4 below.\

### Experimental Example 4

### 4. Experiment for Examining Strength Depending on Content of HPMC (Hydroxypropyl Methylcellulose)

In Experimental Example 3 above, the maximum breaking force (N) immediately before each sphere was broken, that is, the maximum peak value, was measured. The results of the measurement are shown in Table 4 below. It can be interpreted that the greater the maximum breaking force (N), the grater the strength.

**Table 4**

| Content (parts by weight) | Long-axis length (mm) | Short-axis length (mm) | Maximum breaking force | | Short-axis length (mm) after change | Short-axis change rate (%) |
|---|---|---|---|---|---|---|
| | | | (kgf) | (N) | | |
| 0.1 | 8.32 | 8.18 | 1.04 | 10.18 | 3.53 | 43.15 |
| 0.2 | 8.85 | 8.56 | 1.37 | 13.39 | 3.61 | 42.17 |
| 0.3 | 8.72 | 8.53 | 2.32 | 22.69 | 3.36 | 42.56 |
| 0.4 | 10.43 | 10.31 | 2.43 | 23.84 | 3.65 | 35.40 |
| 0.6 | 10.08 | 9.39 | 3.36 | 32.93 | 4.01 | 42.71 |
| 0.8 | 12.27 | 11.32 | 2.95 | 28.94 | 4.36 | 38.52 |
| 1.2 | 11.48 | 10.65 | 3.36 | 32.90 | 4.80 | 45.07 |
| 1.5 | 12.88 | 12.60 | 3.06 | 30.01 | 5.36 | 42.54 |
| 2 | 13.15 | 13.03 | 3.45 | 33.80 | 6.42 | 49.27 |
| 3 | 16.50 | 14.90 | 3.55 | 34.75 | 9.45 | 63.42 |
| 4 | 17.43 | 17.03 | 2.63 | 25.74 | 10.58 | 62.13 |
| 5 | 18.51 | 18.11 | 1.89 | 18.52 | 11.15 | 61.57 |
| 6 | 19.10 | 18.47 | 1.33 | 13.08 | 12.10 | 65.51 |

### Experimental Example 5

### 5. Examination of Shape Retainability Using Maximum Breaking Force (N) Relative to Short-Axis Change Rate

In order to form a bone graft composition having excellent shape retainability, optimal strength and non-resilience are required. Table 5 below shows the maximum breaking force relative to the short-axis change rate depending on the content of HPMC (hydroxypropyl methylcellulose). FIG. 4 shows "maximum breaking force (N) of bone graft composition sphere/short-axis change rate of bone graft composition sphere" as a function of the content (parts by weight) of hydroxypropyl methylcellulose, calculated based on the results shown in Table 5.

**Table 5**

| Content (parts by weight) | Maximum breaking force (N) / short-axis change rate |
|---|---|
| 0.1 | 23.6 |
| 0.2 | 31.8 |
| 0.3 | 53.3 |
| 0.4 | 67.3 |
| 0.6 | 77.1 |
| 0.8 | 75.1 |
| 1.2 | 73.0 |
| 1.5 | 70.5 |
| 2 | 68.6 |
| 3 | 54.8 |
| 4 | 41.4 |
| 5 | 30.1 |
| 6 | 20.0 |

When the content of hydroxypropyl methylcellulose (HPMC) was 0.1 to 0.2 parts by weight, the short-axis change rate was high and the maximum breaking force (N) was low. Thus, in this case, the "maximum breaking force (N)/short-axis change rate" value was low, and hence the shape retainability was not excellent. This means that the shape is easily changed even with a slight force, and this change is very unfavorable during a medical procedure for application of the bone graft composition. This is understood as a phenomenon occurring because the amount of the HPMC capable of increasing the stiffness of the bone graft composition is small.

In addition, in the case of each of the bone graft compositions containing 4 parts by weight or more of hydroxypropyl methylcellulose (HPMC), the short-axis change rate was high and the maximum breaking force (N) was low. Thus, in this case, the "maximum breaking force (N)/short-axis change rate" value was low, and hence the shape retainability was not excellent. That is, it can be seen that, when the content of HPMC in the bone graft composition is excessively high (4 parts by weight or more), the HPMC decreases the shape retainability of the bone graft composition without increasing the stiffness of the bone graft composition.

When hydroxypropyl methylcellulose (HPMC) was contained in an amount of 0.3 to 3 parts by weight based on 1 part by weight of the bone graft material, it is possible to obtain a bone graft composition having excellent shape retainability due to a "maximum breaking force (N)/short-axis change rate" of 50 or more. In another example, when hydroxypropyl methylcellulose (HPMC) was contained in an amount of 0.4 to 2 parts by weight based on 1 part by weight of the bone graft material, it is possible to obtain a bone graft composition having excellent shape retainability due to a "maximum breaking force (N)/short-axis change rate" of 60 or more. In still another example, when hydroxypropyl methylcellulose (HPMC) was contained in an amount of 0.6 to 1.5 parts by weight based on 1 part by weight of the bone graft material, it is possible to obtain a bone graft composition having excellent shape retainability due to a "maximum breaking force (N)/short-axis change rate" of 70 or more.

### Experimental Example 6

### 6. Measurement of Osmotic Pressure of Saline

An osmotic pressure refers to a pressure that is applied to semipermeable membrane when an osmotic phenomenon occurs, and it is proportional to the difference in the solution concentration. In a control experiment, the osmotic pressure of saline was measured, and as shown in Table 6 below, whether the osmotic pressure changed over time was also measured. The osmotic pressure can be obtained by introducing a solution and measuring the osmotic pressure value by a pressure sensor connected to an outlet. The osmotic pressure value of saline measured in this control experiment was 286, which was set to 100% as a reference value.

### Experimental Example 7

### 7. Preparation of Hydrated Bone Graft Materials Containing Hydroxypropyl Methylcellulose (HPMC)

Hydrated bone graft materials containing hydroxypropyl methylcellulose (HPMC) were prepared by adding water to 1 part by weight (0.5 g) of a bone graft material and 0.6 parts by weight of HPMC. The amount of water added was 0.4 to 6 parts by weight based on 1 part by weight of the mixture of the bone graft material and HPMC.

### Experimental Example 8

### 8. Measurement of Osmotic Pressure of Saline Added to Hydrated Bone Graft Materials Containing Hydroxypropyl Methylcellulose (HPMC)

Each of the hydrated bone graft materials prepared in the above section 7 was placed in a 15-ml conical tube, and 5 ml of saline was added thereto. At each of the time points shown in Table 6 below, 0.5 ml of the saline was taken and the osmotic pressure was measured. The measured osmotic pressure value was expressed as a percentage (%) relative to the reference value obtained in the control experiment of the above section 6.

**Table 6**

| | 1 hour | 3 hours | 6 hours | 12 hours | 24 hours | 48 hours |
|---|---|---|---|---|---|---|
| Control | 100% | 100% | 100% | 100% | 100% | 100% |
| 0.4 | 100% | 108% | 112% | 113% | 115% | 117% |
| 0.5 | 100% | 104% | 109% | 110% | 111% | 112% |
| 0.6 | 100% | 103% | 107% | 109% | 110% | 111% |
| 0.8 | 100% | 103% | 106% | 107% | 108% | 109% |
| 1.0 | 100% | 102% | 103% | 106% | 107% | 108% |
| 1.2 | 100% | 103% | 104% | 105% | 106% | 108% |
| 1.5 | 100% | 101% | 102% | 105% | 107% | 108% |
| 2.0 | 100% | 102% | 102% | 104% | 106% | 107% |
| 3.0 | 100% | 109% | 115% | 116% | 117% | 117% |
| 4.0 | 100% | 109% | 116% | 117% | 118% | 118% |
| 6.0 | 100% | 110% | 114% | 116% | 118% | 118% |

As shown in Table 6 above, the value obtained in the control experiment was set to 100% as a reference value. This value is a value obtained by standardizing the osmotic pressure of pure saline, measured at each time point.

As shown in Table 6 above, it can be confirmed that when the amount (parts by weight) of water added was constant, the osmotic pressure increased over time. This suggests that the time and amount of contact between the saline and the hydrated bone graft material containing dissolved HPMC increased over time, thus increasing the osmotic pressure.

As shown in Table 6 above, it can be confirmed that, at the same time point, the osmotic pressure increased as the amount (parts by weight) of water added increased. This suggests that, as the amount of water added increased, the HPMC in the hydrated bone graft material containing dissolved HPMC dissolved more quickly, thus increasing the osmotic pressure.

As shown in FIG. 5, it can be confirmed that when the amount of water added was 0.4 parts by weight or less, the osmotic pressure greatly increased within a short time, and thus the osmotic ratio greatly changed, and even when the amount of water added was 3 parts by weight or more, the osmotic pressure greatly increased within a short time, and thus the osmotic ratio greatly changed. The great increase in the osmotic pressure within a short time means that the HPMC was not fused with the bone graft material and did flow out, and thus the osmotic pressure increased with increasing concentration, leading to a decrease in the shape retainability. Therefore, in order to optimize the shape retainability, the amount of water added is preferably set to 0.5 to 2 parts by weight per part by weight of the bone graft composition containing HPMC.

When the amount of water added is 0.5 to 2 parts by weight based on the weight of the bone graft composition, the osmotic pressure of saline may reach 104 to 112% within 12 to 48 hours. It is insufficient to understand performance on the functional maintenance of the bone graft composition from the osmotic pressure value that is reached within 12 hours, and when this value is applied as it is, the demonstration of the functionality and stability as a bone graft material will be somewhat insufficient. Therefore, by examining the osmotic pressure that is reached within 12 to 48 hours, it is possible to determine the functionality, osmotic properties, shape retainability and the like as a bone graft material. If the osmotic pressure within 48 hours exceeds 115% of the osmotic pressure of saline, it means that hydration of the bone graft composition containing HPMC did not occur well. In this case, the water does not sufficiently function as an additive, and thus the composition is difficult to use as a bone graft composition. If the osmotic pressure within 48 hours is close to 100%, it is not different from the osmotic pressure of saline, and thus it is hardly considered that the bone graft composition containing hydroxypropyl methylcellulose (HPMC) has the desired properties and functions. Therefore, it is preferable to prepare a bone graft composition with which saline reaches an osmotic pressure of 104 to 112% of the osmotic pressure, obtained in the control experiment, within 12 hours to 48 hours after addition thereof.

As described above, the bone graft composition containing hydroxypropyl methylcellulose according to the present disclosure shows an excellent resolution rate of hydroxypropyl methylcellulose within a certain time, has excellent shape retainability due to the strong strength and non-resilience thereof, and forms an optimum osmotic pressure with another solution. Thus, the bone graft composition has excellent effects in terms of activation of bone formation, biocompatibility, and ease of use.

**The invention further relates to the following numbered items:**
1. A bone graft composition containing a porous bone graft material and hydroxypropyl methylcellulose, wherein a content of the hydroxypropyl methylcellulose is 0.15 to 6 parts by weight based on 1 part by weight of the porous bone graft material.
2. A bone graft composition containing a porous bone graft material and hydroxypropyl methylcellulose, wherein a content of the hydroxypropyl methylcellulose is 0.2 to 5 parts by weight based on 1 part by weight of the porous bone graft material.
3. A bone graft composition containing a porous bone graft material and hydroxypropyl methylcellulose, wherein a content of the hydroxypropyl methylcellulose is 0.25 to 4 parts by weight based on 1 part by weight of the porous bone graft material.
4. A bone graft composition containing a porous bone graft material and hydroxypropyl methylcellulose, wherein a content of the hydroxypropyl methylcellulose is 0.3 to 3 parts by weight based on 1 part by weight of the porous bone graft material.

## Claims

1. A bone graft composition containing a porous bone graft material and hydroxypropyl methylcellulose,
wherein the bone graft composition reaches an osmotic pressure of 104 to 112% within 12 to 48 hours after the bone graft solution added to saline, and
wherein a content of the hydroxypropyl methylcellulose is 0.15 to 6 parts by weight based on 1 part by weight of the porous bone graft material.

2. A bone graft composition containing a porous bone graft material and hydroxypropyl methylcellulose,
wherein the bone graft composition reaches an osmotic pressure of 104 to 112% within 12 to 48 hours after the bone graft solution added to saline, and
wherein a content of the hydroxypropyl methylcellulose is 0.2 to 5 parts by weight based on 1 part by weight of the porous bone graft material.

3. A bone graft composition containing a porous bone graft material and hydroxypropyl methylcellulose,
wherein the bone graft composition reaches an osmotic pressure of 104 to 112% within 12 to 48 hours after the bone graft solution added to saline, and
wherein a content of the hydroxypropyl methylcellulose is 0.25 to 4 parts by weight based on 1 part by weight of the porous bone graft material.

4. A bone graft composition containing a porous bone graft material and hydroxypropyl methylcellulose,
wherein the bone graft composition reaches an osmotic pressure of 104 to 112% within 12 to 48 hours after the bone graft solution added to saline, and
wherein a content of the hydroxypropyl methylcellulose is 0.3 to 3 parts by weight based on 1 part by weight of the porous bone graft material.
